# EUROPÄISCHE PATENTSCHRIFT

(11) **EP 1 453 975 B1**
(45) Veröffentlichungstag und Bekanntmachung des Hinweises auf die Patenterteilung: **09.08.2006**
(21) Anmeldenummer: 02787794.3
(22) Anmeldetag: 25.11.2002
(51) Int. Cl.: C12Q 1/68

(54) **VERFAHREN ZUM NACHWEIS VON HYBRIDISIERUNGSEREIGNISSEN IN NUKLEINSÄUREN**
METHOD FOR DETECTING HYBRIDIZATION EVENTS IN NUCLEIC ACIDS
PROCEDE DE MISE EN EVIDENCE DE PROCESSUS D'HYBRIDATION DANS DES ACIDES NUCLEIQUES

(30) Priorität: 29.11.2001 DE 10158516
(43) Veröffentlichungstag der Anmeldung: 08.09.2004
(73) Patentinhaber: Focusgenomics GmbH, 14513 Teltow (DE)
(72) Erfinder: CHARLE, Christoph, 10777 Berlin (DE)
(74) Vertreter: Keller, Günter
(86) Internationale Anmeldenummer: PCT/EP2002/013215
(87) Internationale Veröffentlichungsnummer: WO 2003/046215

(56) Entgegenhaltungen:
- US-A- 5 925 517
- TYAGI S ET AL: "MOLECULAR BEACONS: PROBES THAT FLUORESCE UPON HYBRIDIZATION" NATURE BIOTECHNOLOGY, NATURE PUBLISHING, US, Bd. 14, 1. März 1996 (1996-03-01), Seiten 303-308, XP000196024 ISSN: 1087-0156 in der Anmeldung erwähnt

## Beschreibung

Die Erfindung betrifft insbesondere ein Verfahren zum sequenzspezifischen Nachweis von RNA- oder DNA-Molekülen (im folgenden als Ziel-Nukleinsäuren bezeichnet) durch festphasengebundene markierte Oligonukleotide, die im folgenden als Sonden-Nukleinsäuren bezeichnet werden.

Diese Sonden-Nukleinsäuren, die DNA oder DNA/PNA-Chimären sein können, sind dazu in der Lage, auf Grund komplementärer Bereiche in der Basensequenz intramolekulare Sekundärstrukturen auszubilden, die doppelsträngige Bereiche enthalten. Diese doppelsträngigen Bereiche enthalten Sequenzmotive, die von doppelstrangspezifischen Nukleasen (Restriktionsendonukleasen) erkannt und gespalten werden können und hier als Spaltmodule bezeichnet werden. Die Bildung der intramolekularen Spaltmodule innerhalb der Sonden-Nukleinsäuren wird durch Hybridisierung der Sonden-Nukleinsäuren mit komplementären Ziel-Nukleinsäuren aus der zu untersuchenden Probe verhindert, so daß im Anschluß an den Verdau mit doppelstrangspezifischen Nukleasen die hybridisierten Sonden-Nukleinsäuren von den nicht hybridisierten Sonden-Nukleasen unterschieden werden können.

Das Verfahren umfaßt in bevorzugter Ausführungsform den spezifischen Nachweis von verschiedenen Ziel-Nukleinsäuren, deren Basensequenz nicht identisch ist, durch unterschiedlich markierte Sonden-Nukleinsäuren, die alle auf einem Punkt bzw. einer definierten Fläche immobilisiert sind. Die miniaturisierte Anordnung vieler solcher Nukleinsäuren auf kleinster Fläche ist aus der Biochiptechnologie gut bekannt.

Bei gängigen Verfahren zur Herstellung von DNA-Arrays wird eine unmarkierte Sonden-Nukleinsäure durch in situ Oligonukleotidsynthese [Fodor, S.P.A. et al., "Very large scale immobilized polymer synthesis" U.S. Pat. No. 5,424,186], oder Druckverfahren [Cheung, V.G. et al., "Making and reading microarrays", Nature Genetics, vol. 21, Jan. 1999; Bowtell, D.D.L., "Options available - from start to finish - for obtaining expression data by microarray", Nature Genetics, vol. 21, 1999] auf eine feste Matrix gebracht und mit dieser kovalent verknüpft. Die Sonden-Nukleinsäuren sind in Form sogenannter "Spots" auf der Oberfläche des DNA-Arrays organisiert. Es ist nicht möglich, vor einem Hybridisierungsexperiment die Menge der an der festen Matrix immobilisierten Nukleinsäuren zu bestimmen. Erst durch Hybridisierung des DNA-Arrays mit der markierten Proben-Nukleinsäure und einer zweiten, markierten Proben-Nukleinsäure (double labeling, Wang, B., "Quantitative microarray hybridization assays", U.S. Pat. No. 6,004,755), die als interner Standard dient, ist es möglich, Differenzen in der Menge der an der festen Matrix immobilisierten, nicht-markierten Sonden-Nukleinsäuren rechnerisch äuszugleichen.

Ein anderes Verfahren nutzt das Prinzip des Nuklease-Protektions-Tests [Sambrook, J. et al., "Molecular Cloning" 2001, 3rd Edition, Cold Spring Harbor Laboratory], um nicht hybridisierte, also einzelsträngige, markierte Sonden-Nukleinsäuren, mit Hilfe einzelstrangspezifischer Nukleasen abzubauen [Kumar, R. et al., "Nuclease protection assays", U.S. Pat. 5,770,370]. Die Genauigkeit dieser Methode ist vor allem von der Stabilität der aus Sonden- und Ziel-Nukleinsäuren bestehenden Duplices und der Spezifität der eingesetzten einzelstrangspezifischen Nukleasen abhängig. Die Stabilität einer gegebenen Nukleinsäureduplex, die eine DNA/DNA-, DNA/RNA-, DNA/PNA-, RNA/RNA-, RNA/PNA- oder PNA/PNA-Duplex sein kann, ist durch die Anzahl und Stärke der Watson-Crick Basenpaarungen zwischen den komplementären Strängen vorgegeben, die durch Wasserstoffbrückenbindungen vermittelt werden [Lewin, B., "Genes VI". 1997, Oxford University Press (und andere gängige Lehrbücher der Molekularbiologie)]. In den Endbereichen sind Nukleinsäurenduplices dem Einfluß des umgebenden Mediums, H₂O, ausgesetzt, das die Wasserstoffbrücken zwischen den komplementären Strängen schwächt. Unter Bedingungen, die die Reaktion von einzelstrangspezifischen Nukleasen fördern (30 - 37 °C) liegen die Endbereiche der Duplices aus diesem Grund zum Teil einzelsträngig vor und können durch die Nukleasen gespalten werden. Ein weiterer Nachteil dieser Methode ist, daß einzelstrangspezifische Nukleasen wie S1-Nuklease, Mung Bean Nuklease, RNase A, RNase T1, Exonuklease VII, Bal 31 Nuklease, Micrococcus Nuklease oder Nuklease P1 Nukleinsäuren nicht sequenzspezifisch spalten und sehr leicht doppelsträngige Bereiche abbauen, wenn das Mengenverhältnis von Nukleinsäure und Nuklease im Reaktionsgemisch nicht genau titriert ist [Sambrook, J. et al., "Molecular Cloning" 2001, 3rd Edition, Cold Spring Harbor Laboratory].

Ein weiteres System, mit dessen Hilfe die Hybridisierung von nichtmarkierten Proben-Nukleinsäuren nachgewiesen werden kann, setzt sogenannte Molecular Beacons [Tyagi, S. et al., "Detectably labeled dual conformation oligonucleotide probes, assays and kits", U.S. Pat. 5,925,517] als Sonden-Nukleinsäuren ein, die kovalent an Mikro- bzw. Nanopartikel aus Glas gebunden sind [Steemers, F.J. et al., "Screening unlabeled DNA targets with randomly ordered fiber-optic gene arrays", 2000, Nature Biotech., vol. 18]. Molecular Beacons sind Sonden-Nukleinsäuren, die Intramolekulare Sekundärstrukturen ausbilden können und deren Enden kovalent mit verschiedenen Fluorophoren verknüpft sind, die infolge der intramolekularen Sekundärstruktur in enge räumliche Nähe zueinander gebracht werden. Eine der beiden Fluorophoren (Quencher) absorbiert die von der anderen Fluorophore (Emitter) emittierten Photonen. Die Hybridisierung eines Molecular Beacon mit einer Ziel-Nukleinsäure löst die intramolekulare Sekundärstruktur auf und das von der angeregten Fluorophore (Emitter) emittierte Licht kann nicht mehr durch den Quencher absorbiert werden. Dieses Verfahren hat den entscheidenden Nachteil, daß das Signal-Hintergrund Verhältnis ca. 25 : 1 beträgt [Steemers, F.J. et al., "Screening unlabeled DNA targets with randomly ordered fiber-optic gene arrays", 2000, Nature Biotech., vol. 18]. Da die Unterschiede in der Transkriptionsrate verschiedener Gene erheblich größer sein können als das Signal-Hintergrund-Verhältnis von Molecular Beacons, sind Systeme, die auf Basis von Elektronen- bzw. Fluoreszenz-Resonanz-Energie Transfer arbeiten ausschließlich im Bereich der quantitativen Bestimmung von Ziel-Nukleinsäuren durch Amplifikation einer Zielsequenz [Gelfand, D.H. et al.: "Detection of specific polymerase chain reaction product by utilizing the 5' to 3' exonuclease activity of Thermus aquaticus DNA-polymerase", 1991, Proc.Natl.Acad.Sci., vol. 88 and US Pat. 5,210,015 (1993); Tyagi, S. et al.: "Molecular Beacons: probes that fluoresce upon hybridization", 1996, Nature Biotech., vol. 14] oder im Rahmen der nicht quantitativen Bestimmung von Nukleinsäuren [Steemers, F.J. et al., "Screening unlabeled DNA targets with randomly ordered fiber-optic gene arrays", 2000, Nature Biotech., vol. 18].

Auf DNA-Arrays können derzeit mehr als 10.000 "Spots" pro cm², also mehr als 10.000 unterschiedliche Nukleinsäure-Sonden immobilisiert werden [Bowtell, D.D.L., "Options available - from start to finish - for obtaining expression data by microarray", Nature Genetics, vol. 21, Jan. 1999]. Die Höchstzahl voneinander unterscheidbarer Probenpunkte ("Spots") wird durch die kleinste technisch erreichbare Punktgröße festgelegt. Diese ist, da bei der Array-Herstellung Flüssigkeitsmengen im Nanolitermaßstab transferiert werden, von physikalischen Größen wie Viskosität und Oberflächenspannung der transferierten Flüssigkeiten abhängig. Ein weiterer Parameter, der die Größe der Probenpunkte nach unten begrenzt, ist das optische Auflösungsvermögen des Lichtmikroskops, da sämtliche Geräte zur Detektion von Fluoreszenz, Lumineszenz oder Phosphoreszenz mit optischen Systemen arbeiten, die dem eines Lichtmikroskops (Confocal Laser Scanning Microscope) entsprechen. Es ist daher nicht möglich, beliebig viele Probenpunkte ("Spots") auf einem DNA-Array zu fixieren.

Gegenstand der vorliegenden Erfindung ist daher ein Verfahren zum Nachweis von Zielnukleinsäure durch Hybridisierung, wobei in dem Verfahren
a) eine Hybridisierung mit wenigstens einer Sonden-Nukleinsäure durchgeführt wird, die mit einem Ende an einer festen Phase gebunden ist. Jede Sonden-Nukleinsäure weist eine Zielnukleinsäuresequenz auf, die am 3'-Ende von einer kurzen Nukleinsäuresequenz und am 5'-Ende von einer hierzu komplementären Nukleinsäuresequenz flankiert ist, die einen kurzen DNA-Doppelstrang ausbilden können. Dieser kann von einer Doppelstrang-spezifischen Nuklease (Restriktionsendonuklease) gespalten werden und stellt dadurch ein Spaltmodul dar. Weiterhin weist die Sonden-Nukleinsäure am anderen Ende, das nicht an die feste Phase gebunden ist, eine Markierung auf;
b) wenigstens eine Behandlung mit wenigstens einer Doppelstrang-spezifischen Nuklease durchgeführt wird. Durch den Verdau mit der Nuklease werden nur diejenigen Spaltmodule geschnitten, die einen Doppelstrang ausgebildet haben. Wenn sich kein Doppelstrang ausbilden konnte, weil die Ziel-Nukleinsäure mit einer nachzuweisenden Nukleinsäure hybridisiert hat, bildet sich kein doppelsträngiges Spaltmodul und die Nukleinsäure wird nicht geschnitten.
c) Schließlich wird der Anteil der Markierung bestimmt, der nach Schritt b) an der festen Phase gebunden ist.

In bevorzugter Ausführungsform werden in einem Verfahrensansatz mehrere verschiedene Sonden-Nukleinsäuren eingesetzt, die unterschiedliche Zielsequenzen beinhalten.

Bevorzugt ist auch, daß in einem Verfahren mehrere Sonden-Nukleinsäuren eingesetzt werden, die unterschiedliche Spaltmodule aufweisen, die wieder von verschiedenen Doppelstrang-spezifischen Nukleasen gespalten werden können. Ebenso können auch die Sonden-Nukleinsäuren unterschiedliche Markierungen aufweisen, wobei es sich bei den Markierungen um Fluorophore und/oder Teile eines Bindungspaares handelt.

Ein Vorteil des erfindungsgemäßen Verfahrens ist, daß die erfindungsgemäß verwendeten Sonden-Nukleinsäuren unterschiedliche Variablen beinhalten. Einerseits können die Zielsequenzen variiert werden und dadurch können ganz verschiedene Nukleinsäuresequenzen in der zu untersuchenden Probe nachgewiesen werden. Andererseits können die Spaltmodule Erkennungssequenzen für verschiedene Restriktionsendonukleasen beinhalten. Dadurch können die Sonden-Nukleinsäuren entweder parallel oder auch sequenziell mit verschiedenen Restriktionsendonukleasen verdaut werden. Schließlich können die Sonden-Nukleinsäuren auch unterschiedliche Markierungen aufweisen. Je nach Aufgabenstellung kann dann der entsprechende DNA-Array konfiguriert werden und es können mehrere verschiedene Behandlungs- und Auswertungsschritte entweder parallel oder sequenziell durchgeführt werden und es kann hierdurch ein Maximum an Information erhalten werden.

Das erfindungsgemäße Verfahren kann mit einem Kit zum Nachweis von Hybridisierungen mit Zielnukleinsäuren durchgeführt werden, wobei mit dem Kit wenigstens eine Hybridisierung mit wenigstens einer erfindungsgemäß verwendeten Sonden-Nukleinsäure durchgeführt werden kann.

Im Rahmen des erfindungsgemäßen Verfahrens können auch Techniken, die aus anderen Nukleinsäure-Nachweisverfahren bekannt sind, eingesetzt werden. Die sequenzspezifische Detektion von Ziel-Nukleinsäuren, die DNA oder RNA sein können, erfolgt bei Verfahren wie Northemblots, Southemblots oder Nuklease Protection Assays durch den Nachweis der Bildung von Hybriden (Duplices) aus Ziel-Nukleinsäuren und markierten Sonden-Nukleinsäuren, die DNA, RNA oder PNA sein können. Bei DNA-Arrays liegt die unmarkierte Sonden-Nukleinsäure an eine feste Matrix gebunden vor und wird mit markierter cDNA oder cRNA (im folgenden als Proben-Nukleinsäuren bezeichnet) hybridisiert. Im allgemeinen werden Hybridisierungsereignisse in Nukleinsäuren durch den Nachweis von Fluoreszenz, Chemiluminseszenz, Chemifluoreszenz oder Radioaktivität nachgewiesen [Sambrook, J. et al., "Molecular Cloning" 2001, 3rd Edition, Cold Spring Harbor Laboratory]. Zur Fluoreszenzmarkierung von Proben- bzw. Sonden-Nukleinsäuren können eine Reihe von Fluorophoren, wie z.B. Fluorescein, Lissamin, Phycoerythrin, Rhodamin (Perkin Eimer Cetus), Cy2, Cy3, Cy 3.5, Cy5, Cy5.5, Cy7, FluorX (Amersham), eingesetzt werden [Kricka; L.: "Non isotopic DNA probe techniques", 1992, Academic Press, San Diego]. Zur Markierung von Nukleinsäuren können neben den hier aufgeführten Fluorophoren auch andere, hier nicht erwähnte Fluorophoren eingesetzt werden. Diese umfassen sämtliche Fluorophoren, die kovalent mit Nukleinsäuren verknüpft werden können und deren Anregungs- und Emissionsmaxima im Infrarotbereich, im sichtbaren Bereich oder im UV-Bereich des Spektrums liegen. Werden Proben- bzw. Sonden Nukleinsäure mit Teilen eines Bindungspaares wie Biotin, Digoxigenin oder anderen Haptenen markiert, wird nach Hybridisierung der mit einer detektierbaren Markierung konjugierte zweite Teil des Bindungspaares (Streptavidin oder anti-Digoxigenin AK) mit den Duplices inkubiert. Die detektierbare Markierung des zweiten Teils des Bindungspaares kann eine Fluorophore oder ein Enzym (alkalische Phosphatase, Horseradish Peroxidase u.a.) sein, das ein Substrat unter Lichtemission (Chemiluminseszenz oder Chemifluoreszenz) umsetzt ["Fluorescent and Luminescent Probes for biological activity", 1999, 2nd Edition. Mason, W.T. ed.].

Die nicht-radioaktive Markierung von Nukleinsäuren erfolgt durch die enzymkatalysierte Synthese von DNA oder RNA in Anwesenheit von Nukleotid-Triphosphaten, deren Nukleotidbasen kovalent mit Fluorophoren, Teilen eines Bindungspaares (z.B. Biotin, Digoxigenin oder anderen Haptene) oder reaktiven Gruppen (NH₂ oder SH) verknüpft sind. Im Verlauf der Synthese, die durch DNA- bzw. RNA-Polymerasen (AMV-Reverse Transkriptase, MMuLV-Reverse Transkriptase, T7-RNA-Polymerase, T3-RNA-Polymerase, SP6-RNA-Polymerase, Taq-Polymerase, Klenow-Fragment, DNA-Polymerase und andere katalysiert wird, werden diese modifizierten Nukleotid-Triphosphate in die neu entstehende Nukleinsäure inkorporiert [Sambrook, J. et al., "Molecular Cloning" 2001, 3rd Edition, Cold Spring Harbor Laboratory ]. Die Integrität der mRNA, die als Matrize zur Synthese der markierten Proben-Nukleinsäuren eingesetzt wird, ist hier von entscheidender Bedeutung. Die Länge einer Sonden-Nukleinsäure, die durch reverse Transkription aus RNA, durch PCR oder durch in vitro Transkription aus DNA synthetisiert wird ist. neben ihrer Basenzusammensetzung, wesentlich für die Markierungseffizienz. Je kürzer eine zu markierende Nukleinsäure ist, desto weniger mit nachweisbaren Gruppen modifizierte Nukleotid-Triphosphate (dNTPs oder rNTPs) werden während der Synthese inkorporiert. Wird eine mRNA Population durch RNasen degradiert, so ist die aus dieser RNA-Population synthetisierte Proben-Nukleinsäure zum einen nur sehr schwach markiert und zum anderen nicht repräsentativ für den in einem zu untersuchenden Zelltyp, Gewebe oder Organismus vorherrschenden Transkriptionszustand. Eine repräsentative Proben-Nukleinsäure kann nur aus absolut intakter mRNA synthetisiert werden. Nukleotid-Triphosphate, deren Basen mit den oben aufgeführten Gruppen modifiziert wurden, werden von sämtlichen bekannten DNA- bzw. RNA-Polymerasen, welche die Synthese von DNA bzw. RNA katalysieren, mit einer erheblich geringeren Effizienz als nicht modifizierte Nukleotid-Triphosphate inkorporiert [Molecular Dynamics Inc., "Fluorescent DNA-Labeling by PCR", 1999, Molecular Dynamics Application Note # 62]. So ist die Ausbeute an markierter Sonden-Nukleinsäure, verglichen mit radioaktiven Systemen, gering und der Verlust an Probenmaterial durch die der Synthese folgenden Reinigungsschritte sehr hoch.

Zur Synthese nicht-radioaktiv markierter Nukleinsäuren, die z.B. zur Hybridisierung von DNA-Arrays eingesetzt werden, müssen aus diesem Grund relativ große Mengen an Probenmaterial, wie z.B. Kulturzellen oder Gewebeproben, aus dem die zu analysierende mRNA isoliert wird, eingesetzt werden. Daher sind DNA-Arrays in Bereichen wie z. B. der klinischen Diagnostik, in denen die Menge des für die Analyse zur Verfügung stehenden Probenmaterials begrenzt ist, nur eingeschränkt einsetzbar. Des weiteren haben Nukleinsäuren die Eigenschaft, sich an der Oberfläche von Schmutzpartikeln anzulagern. Die Detektion festphasengebundener nichtmarkierter Nukleinsäuren durch Hybridisierung mit markierten Nukleinsäuren kann aus diesem Grund, vor allem auf DNA-Arrays, durch Schmutzpartikel empfindlich gestört werden und so zu falsch-positiven Ergebnissen führen.

Im Bereich der Biotechnologie und vor allem in Bereichen wie der klinischen Diagnostik oder der industriellen Wirkstoff-Forschung, die auf automatisierte Methoden angewiesen sind, um eine hohen Probendurchsatz zu gewährleisten, besteht ein Bedarf an Methoden zur Durchführung von Expressionsanalysen, die eine Standardisierung der Meßergebnisse ohne aufwendige Kalibrierungsmessungen ermöglichen. Dem Stand der Technik entsprechende Systeme zum Nachweis von Hybridisierungsereignissen in Nukleinsäuren, die im Bereich der Expressionsanalyse eingesetzt werden, sind stark von der Integrität der zu untersuchenden mRNA abhängig, die als Proben-Nukleinsäure bzw. als Matrize zur Synthese von Proben-Nukleinsäuren eingesetzt wird. Diese Systeme sind vor allem auf Grund der Markierung der zu hybridisierenden, nicht festphasengebundenen Nukleinsäuren (Proben-Nukleinsäuren) anfällig für Fehlinterpretationen, welche die gesamte Messung verfälschen können.

Das erfindungsgemäße Verfahren kann verwendet werden zu Expressionsanalysen, in denen die sequenzspezifische Hybridisierung von unmarkierter Ziel-Nukleinsäure mit festphasengebundenen Sonden unabhängig von der Integrität der Proben-Nukleinsäuren quantitativ bestimmt werden kann. Während die maximale Anzahl der auf einer Oberfläche nachweisbaren verschiedenen Sonden-Nukleinsäuren durch physikalische Grenzen im Bereich der Optik und der "Microfluidics" limitiert ist, wird erfindungsgemäß ein DNA-Array mit einem System offenbart, das, nicht durch die physikalischen Grenzen im Bereich der Optik und der "Microfluidics" limitiert, gegenüber dem Stand der Technik entsprechenden Systemen die Menge der auf einer definierten Fläche nachweisbaren Sonden-Nukleinsäuren deutlich erhöht werden kann.

In einer bevorzugten Ausführungsform wird ein Verfahren zum Nachweis von Hybridisierungsereignissen an festphasengebundenen Sonden-Nukleinsäuren offenbart, das durch Verwendung festphasengebundener markierter Sonden die Standardisierung der Meßergebnisse, den Einsatz von unmarkierter Proben- bzw. Ziel-Nukleinsäuren und die simultane Analyse der Expression verschiedener Ziel-Nukleinsäuren auf einem definierten Punkt ermöglicht. Die erfindungsgemäßen, festphasengebundenen Sonden-Nukleinsäuren, die DNA oder DNA/PNA-Chimären [Finn, P.J. et al.: "Synthesis and properties of DNA-PNA chimeric oligomers", 1996, Nuc.Acids.Res., vol. 24 (17); Ratilainen, T. et al.: "Thermodynamics of sequence-specific binding of PNA to DNA", 2000, Biochemistry, vol. 39; van der Laan, A.C. et al.: "Optimization of the binding properties of PNA-(5')-DNA-Chimerae", 1998, Bioorg.Med.Chem.Lett., vol. 8] sein können, bilden auf Grund ihrer Sequenz intramolekulare Sekundärstrukturen aus, die durch doppelstrangspezifische Endonukleasen erkannt und gespalten werden können. Der Teil der erfindungsgemäßen Sonden-Nukleinsäuren, der durch die Ausbildung der intramolekularen Sekundärstruktur doppelsträngig vorliegt, ist grundsätzlich DNA, um die Zugänglichkeit für doppelstrangspezifische Endonukleasen sicherzustellen.

Durch Hybridisierung der erfindungsgemäßen, festphasengebundenen Sonden-Ziel-Nukleinsäuren mit nachzuweisenden Nukleinsäuren wird die intramolekulare Sekundärstruktur und damit der doppelsträngige Bereich aufgelöst und kann nicht mehr durch doppelstrangspezifische Endonukleasen erkannt und gespalten werden. Nichthybridisierte, festphasengebundene Sonden-Nukleotide werden enzymatisch gespalten. Der markierte Teil der erfindungsgemäßen Sonden-Nukleinsäuren wird durch die enzymatische Spaltung von der Oberfläche abgetrennt und diffundiert in das umgebende Medium und kann gegebenenfalls ausgewaschen werden. Im Anschluß an den enzymatischen Abbau der nichthybridisierten Sonden-Nukleinsäuren wird die Fluoreszenz der hybridisierten, nicht enzymatisch abgeschnittenen Sonden-Nukleinsäuren gemessen. Das Signal-Hintergrund Verhältnis der Methode ist ausschließlich von der Qualität der eingesetzten doppelstrangspezifischen Endonukleasen und der Vollständigkeit der Abtrennung der nichthybridisierten Sonden-Nukleinsäuren abhängig und entspricht dem einer Hybridisierung mit radioaktiv markierten Nukleinsäuren.

Da nicht eine bestimmte Proben- bzw. Ziel-Nukleinsäure markiert wird, sondern die festphasengebundenen Sonden-Nukleinsäuren, können auf einer definierten Fläche bzw. einem Punkt auf einer Oberfläche (Spot) so viele Sonden-Nukleinsäuren unterschiedlicher Sequenzspezifität immobilisiert werden, wie es Fluorophoren gibt, die sich hinsichtlich ihrer Anregungs- bzw. Emissionsmaxima spektral unterscheiden lassen. Die Empfindlichkeit der vorliegenden Methode kann durch die Anzahl der mit der Sonden-Nukleinsäure kovalent verbundenen Fluorophoren beliebig gesteigert werden.

Es können neben den bekannten Fluorophoren auch Teile eines Bindungspaares wie Digoxigenin, Biotin oder andere Haptene zur Markierung der festphasengebundenen Sonden eingesetzt werden. Die Moleküle, welche die verschiedenen Haptene spezifisch binden (Immunglobuline, Streptavidin), sind mit Enzymen unterschiedlicher Substratspezifität kovalent verbunden. Diese Enzyme können Alkalische Phosphatase, Peroxidase, saure Phosphatase und andere sein. So können, abhängig von der Anzahl der verfügbaren unterschiedlichen Haptene und der Anzahl der verfügbaren unterschiedlichen Enzymkonjugate beliebig viele Sonden-Nukleinsäuren unterschiedlicher Sequenzspezifität auf einer definierten Fläche bzw. einem Punkt auf einer Oberfläche (Spot) immobilisiert werden.

Die zur Hybridisierung mit den immobilisierten Sonden-Nukleinsäuren eingesetzten Proben- bzw. Ziel-Nukleinsäuren können nichtmarkierte DNA, cDNA, cRNA oder mRNA sein. Im Gegensatz zu konventionellen Systemen muß zur Hybridisierung nur der Anteil der Ziel- bzw. Proben-Nukleinsäure eingesetzt werden, der komplementär zum Detektionsmodul der Sonden-Nukleinsäure ist, da beim vorliegenden System die Sonden-Nukleinsäure markiert ist. Ein weiterer Vorteil des beschriebenen Systems ist, daß die Sensitivität des Nachweises nicht von der Markierungseffizienz der Proben-Nukleinsäuren abhängig ist, sondern ausschließlich von der Markierung der Sonden-Nukleinsäure. Diese kann aber sehr viel genauer bestimmt werden.

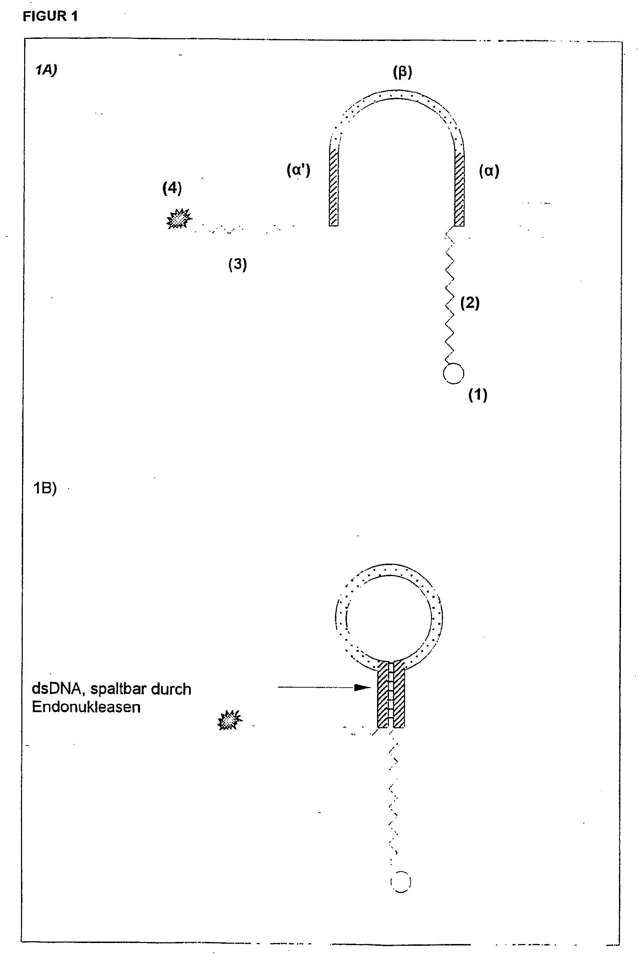

Eine erfindungsgemäße Sonden-Nukleinsäure ist schematisch in Figur 1 A dargestellt. Eine typische Sonden-Nukleinsäure weist folgende Komponenten auf:
- Wenigstens eine funktionelle Gruppe (1) wie z.B. eine Aminogruppe (NH2), Thiolgruppe (SH), oder ein Teil eines Bindungspaares, wie z.B. Biotin, Digoxigenin zur Anbindung an eine feste Phase.
- Ein Spacer-Modul (2) von bevorzugt mehr als 11 C2 Bindungen Länge, dessen eines Ende kovalent mit der funktionellen Gruppe verbunden ist.
- Einen Sequenzabschnitt α (Erkennungs- und Spaltmodul), der eine Länge von bevorzugt 5 - 12 Nukleotiden hat, aus DNA besteht und dessen 3'-Ende kovalent mit dem Ende des Spacer-Moduls verbunden ist, das mit der festen Phase verbunden ist. Sequenzabschnitt α enthält die Erkennungssequenz für eine Restriktionsendonuklease.
- Einen Sequenzabschnitt β (Detektionsmodul), der eine Länge von bevorzugt 12 bis 30 Nukleotiden hat, dessen 3'-Ende kovalent mit dem 5'-Ende von Sequenzabschnitt α (Erkennungs- und Spaltmodul) verknüpft ist und DNA, RNA oder PNA sein kann. Sequenzabschnitt β stellt den Anteil des Moleküls dar, der als Sonde mit einer nachzuweisenden Ziel-Nukleinsäure unter geeigneten Reaktionsbedingungen eine Heteroduplex bilden kann. Die Nukleotide und/oder das Zucker-Phosphat - bzw. Pseudopeptidrückgrat von Sequenzabschnitt β können kovalent mit Fluorophoren verbunden sein.
- Einen Sequenzabschnitt α' (Erkennungs- und Spaltmodul), der aus DNA besteht und dessen 3'-Ende kovalent mit dem 5'-Ende von Sequenzabschnitt β verknüpft ist. Die Sequenz des Abschnitts α' entspricht der revers komplementären Sequenz von Sequenzabschnitt α.
- Ein Spacer-Modul (3) von bevorzugt mehr als 11 C2 Bindungen Länge, das kovalent mit dem 5'-Ende von Sequenzabschnitt α' verbunden ist.
- Gegebenenfalls ein Verzweigungsmodul [Newcome, G.R. et al.: "Dendritic Molecules: Concepts, Synthesis, Perspectives", 1996, VCH Publishers] (nicht dargestellt) das kovalent mit dem Ende von Spacer-Modul (3) verbunden ist, das nicht mit dem 5'-Ende von Sequenzabschnitt α' verbunden ist. An die einzelnen Enden dieses Verzweigungsmoduls können bis zu n weitere Verzweigungsmodule gebunden sein, so daß sich eine maximale Zahl von 3ⁿ Enden ergibt, wobei an jedes Ende
- ein Fluorophor (4) oder Teile eines Bindungspaares (Biotin oder Digoxigenin) gebunden sind.

Die Sonde wird über Element (1) an eine festen Matrix gebunden. Diese feste Oberfläche kann unter anderem eine plane Oberfläche, die auch konvex oder konkav sein kann, eine Faser oder ein Micro- bzw. Nanopartikel aus anorganischem oder organischem Material sein. Die an eine solche feste Matrix gebundenen, erfindungsgemäßen Sonden-Nukleinsäuren werden im folgenden als DNA-Array bezeichnet. Sequenzabschnitt α und α' sind zueinander komplementär und können unter geeigneten Bedingungen einen doppelsträngigen Bereich, die Duplex α-α' ausbilden (siehe Figur 1 B). Die Ausbildung einer Haarnadelstruktur durch intramolekulare Duplexformation ist für dieses Molekül gegenüber der einzelsträngigen Konformation thermodynamisch begünstigt. So liegt bei einer Temperatur, die niedriger als die Gleichgewichts-Schmelztemperatur Tₘ des Sequenzabschnitts α bzw. α' ist, das Molekül ausschließlich als Haarnadelstruktur mit intramolekularen Duplex α-α' vor, die durch Restriktionsendonukleasen sequenzspezifisch gespalten werden kann.

Nach Spaltung durch eine Restriktionsendonuklease bleiben die Elemente (1), (2) und gegebenenfalls wenige Nukleotide des Elementes (α) an die Festphase gebunden. Markierte Elemente der Sonde diffundieren in das umgebende Medium und werden gegebenenfalls durch Waschen entfernt.

Durch Hybridisierung mit einer Ziel-Nukleinsäure, die RNA oder DNA sein kann und deren Sequenz komplementär zur Sequenz von Sequenzabschnitt β ist, liegt die Sonde partiell als Duplex mit der Proben-Nukleinsäure vor. In diesem Fall liegen die Sequenzabschnitte α und α' einzelsträngig vor und können nicht durch Restriktionsendonukleasen oder andere doppelstrangspezifische Nukleasen gespalten werden. Um eine ausreichende Stabilität der aus Proben-Nukleinsäure und Sequenzabschnitt β bestehenden Heteroduplex gegenüber der intramolekularen Duplex α-α' zu gewährleisten, muß die Gleichgewichts-Schmelztemperatur Tₘ(β) von Sequenzabschnitt β höher sein als die des Sequenzabschnitts α bzw. α'; also Tₘ(β) > Tₘ(α) [Bonnet, G. et al., "Thermodynamic basis of the enhanced specificity of structured DNA probes", 1999, Proc.Natl.Acad.Sci., vol. 96]. Idealerweise ist die Gleichgewichts-Schmelztemperatur Tₘ(β) der aus Proben-Nukleinsäure und Sequenzabschnitt β gebildeten Heteroduplex zwischen 10°C und 25°C höher als die Gleichgewichts-Schmelztemperatur Tₘ(α) der intramolekularen Duplex α-α'. Eine Sonden-Nukleinsäure, die eine Sekundärstruktur ausbilden kann, hat gegenüber einer linearen Sonden-Nukleinsäure mit der gleichen Ziel-Nukleinsäure eine erhöhte Sequenzspezifität. Der Unterschied in der Gleichgewichts-Schmelztemperatur △Tₘ einer Sonden-/Ziel-Nukleinsäurenduplex, die keine Basenfehlpaarung aufweist, gegenüber einer Sonden-/Ziel-Nukleinsäurenduplex die eine Basenfehlpaarung aufweist, ist bei Sonden-Nukleinsäuren, die eine Sekundärstruktur ausbilden können, etwa doppelt so hoch wie bei linearen Sonden-Nukleinsäuren [Bonnet, G. et al., "Thermodynamic basis of the enhanced specificity of structured DNA probes", 1999, Proc.Natl.Acad.Sci., vol. 96].

Das erfindungsgemäße Verfahren kann bevorzugt eingesetzt werden für Multiplexanalysen. Hierbei werden n verschiedene Sonden-Nukleinsäuren, die sich voneinander in Sequenzabschnitt β, im Anregungs- und Emissionsspektrum der Fluorophoren [Vet, J.A.M. et al., "Multiplex detection of four pathogenic retroviruses using molecular beacons", 1999, Proc.Natl.Acad.Sci., vol. 96; Marras, S.A.E. et al., "Multiplex detection of single-nucleotide variations using molecular beacons", 1999, Genetic Analysis; Biomolecular Engineering, vol. 14] und gegebenenfalls bezüglich der in Sequenzabschnitt α und α' enthaltenen Erkennungssequenzen für Restriktionsendonukleasen unterscheiden, auf *derselben* Fläche immobilisiert. Eine wässrige Lösung, die äquimolare Mengen dieser n verschiedenen Sonden-Nukleinsäuren enthält, wird durch Druckverfahren auf einer festen Oberfläche deponiert und immobilisiert [Cheung, V.G. et al., "Making and reading microarrays", Nature Genetics, vol. 21, Jan. 1999; Bowtell, D.D.L., "Options available - from start to finish - for obtaining expression data by microarray", Nature Genetics, vol. 21, Jan. 1999]. Diese Fläche kann ein "Spot" auf einem DNA-Array oder die Oberfläche eines Micro- bzw. Nanopartikels sein.

Die Hybridisierung von n verschiedenen Ziel-Nukleinsäuren unterschiedlicher Sequenz mit n verschiedenen Sonden-Nukleinsäuren, deren Sequenzabschnitte α und α' dieselbe Erkennungssequenz für eine Restriktionsendonuklease enthalten, kann auf diese Weise simultan analysiert werden. Enthalten die Sequenzabschnitte α und α' der Sonden-Nukleinsäuren n verschiedene Erkennungssequenzen für n verschiedene Restriktionsendonukleasen, so kann die Hybridisierung von n verschiedenen Ziel-Nukleinsäuren unterschiedlicher Sequenz mit n verschiedenen Sonden-Nukleinsäuren simultan oder bevorzugt seriell analysiert werden.

Das erfindungsgemäße Verfahren kann zur Durchführung einer Multiplexanalyse zur Hybridisierung der Sonden-Nukleinsäure mit Ziel- bzw. Proben-Nukleinsäuren folgendermaßen durchgeführt werden:

Ein DNA-Array aus einer- oder mehreren an eine feste Matrix gebundenen erfindungsgemäßen Sonden-Nukleinsäuren wird mit unmarkierter Proben-Nukleinsäure, die RNA- oder DNA sein kann, unter den im folgenden aufgeführten bevorzugten Bedingungen in Kontakt gebracht. Der DNA-Array wird entsprechend der zu hybridisierenden Oberfläche mit 20 µl - 200 µl eines geeigneten Hybridisierungspuffers bei 45°C für 10 - 20 Minuten inkubiert.

0,1 µg - 50 µg unmarkierte Proben-Nukleinsäure werden in 300 µl geeigneter Hybridisierungslösung aufgenommen und vor der Hybridisierung mit dem DNA-Array für 5 Minuten auf etwa 99°C erhitzt und anschließend für 5 Minuten auf etwa 45°C abgekühlt. Der Hybridisierungspuffer wird von dem DNA-Array entfernt und durch die die Proben-Nukleinsäuren enthaltende Hybridisierungslösung ersetzt. Der DNA-Array wird 16 Stunden bei 45°C - 60°C mit den Proben-Nukleinsäuren inkubiert. Nach Entfernung der Proben-Nukleinsäurenlösung wird der DNA-Array mit Waschpuffern unterschiedlicher lonenstärke für jeweils bei 50°C - 65°C gewaschen. Die Auswahl der geeigneten Hybridisierungs- und Waschbedingungen richtet sich nach der Art der Proben-Nukleinsäure (DNA oder RNA), ihrer Länge sowie nach Art (DNA, RNA oder PNA) und Länge der immobilisierten Sonden-Nukleinsäuren [Anderson, M.L.M.: "Nucleic acid Hybridization", 1998, Springer-Verlag Telos; Schena, M.: "DNA-Microarrays: A practical approach", 1999, Oxford University Press].

Die Spaltung der nichthybridisierten Sonden-Nukleinsäuren durch Restriktionsendonukleasen im Bereich der Duplex α-α' erfolgt bevorzugt bei 25°C - 37°C unter den vom Hersteller empfohlenen Reaktionsbedingungen. Die Aktivität der Restriktionsendonukleasen kann durch Zugabe von Lipiden zum Reaktionsansatz bis zu 34-fach gesteigert werden [Kinnunen et al., "Materials and methods for digestion of DNA or RNA using restriction endonucleases", U.S. Pat. 5,879,950]. Nach den Waschschritten wird der DNA-Array entsprechend der zu hybridisierenden Oberfläche mit 20 µl - 200 µl des vom Hersteller empfohlenen Reaktionspuffers, der 0,5 U - 5 U der Restriktionsendonuklease enthält, welche die Sonden-Nukleinsäure im Bereich der Duplex α-α' spaltet, für 20 - 60 Minuten bei 25°C - 37°C inkubiert. Durch Waschen mit einem 1X TE-Puffer bei Raumtemperatur werden abgespaltene Proben-Nukleinsäuren, Markierungen und Restriktionsendonukleasen von der Oberfläche des DNA-Arrays entfernt.

Die durch das erfindungsgemäße Verfahren erhaltenen Signale und Daten werden bevorzugt folgendermaßen detektiert und analysiert:

Vor dem Transfer und der Immobilisierung der markierten Sonden auf einer festen Oberfläche durch Druckverfahren [Cheung, V.G. et al., "Making and reading microarrays", Nature Genetics, vol. 21, Jan. 1999; Bowtell,- D.D.L., "Options available - from start to finish - for obtaining expression data by microarray", Nature Genetics, vol. 21, Jan. 1999] wird der Markierungsgrad jeder Sonden-Nukleinsäure bestimmt. Die Nukleinsäurekonzentration wird durch Messung der Absorption einer wässrigen Nukleinsäure-Lösung bei einer Wellenlänge von 260 nm mit Hilfe des Lambert-Beerschen Gesetzes bestimmt (A₂₆₀ = ε x d x c, wobei A₂₆₀ die Absorption bei λ = 260 nm ist, ε der molare Extinktionskoeffizient [cm⁻¹M⁻¹] der Nukleinsäure, der von Basensequenz und Länge der zu untersuchenden Nukleinsäure abhängig ist, d die Schichtdicke der verwendeten Cuvette und c die Konzentration [M] der Nukleinsäure).

Die Konzentration der mit der Sonden-Nukleinsäure konjugierten Fluorophoren wird durch Messung der Absorption einer wässrigen Lösung der markierten Sonden-Nukleinsäuren bei einer Wellenlänge, die dem Absorptionsmaximum der Fluorophore (λₘₐₓ) entspricht, mit Hilfe des Lambert-Beerschen Gesetzes bestimmt. Um eine präzise Bestimmung der Konzentration einer mit Fluorophoren konjugierten Nukleinsäure durchführen zu können, muß berücksichtigt werden, daß die meisten Fluorophoren Licht einer Wellenlänge von 260 nm absorbieren. Von der Gesamtabsorption bei 260 nm wird der Anteil der Fluorophoren subtrahiert, der ein Produkt aus der Absorption der Fluorophore bei der Wellenlänge, die ihrem Absorptionsmaximum (λₘₐₓ) entspricht und einem Korrekturfaktor CF₂₆₀ ist (A_{Nukleinsäure}= A₂₆₀ - (A_{λmax} x CF₂₆₀)). Die molaren Extinktionskoeffizienten verschiedener Fluorophoren und die Korrekturfaktoren für die Absorption bei 260 nm (CF₂₆₀) sind bei den Herstellern dieser Fluorophoren erhältlich (Molecular Probes, BioRad und andere).

Das Verhältnis der Konzentration der mit der Sonden-Nukleinsäure konjugierten Fluorophoren zur Konzentration der Sonden-Nukleinsäure ist gleich der Menge der mit der Sonden-Nukleinsäure konjugierten Fluorophoren. Um die spezifische Fluoreszenz der markierten Sonden-Nukleinsäuren zu bestimmen, wird die Fluoreszenz einer festgelegten Menge dieser Sonden-Nukleinsäuren bestimmt. Anhand der spezifischen Fluoreszenz kann die Anzahl der an eine feste Matrix gebundenen Sonden-Nukleinsäuren-Moleküle vor einer Hybridisierung mit Ziel- bzw. Proben-Nukleinsäure präzise bestimmt werden. Schwankungen in der Menge der immobilisierten Sonden-Nukleinsäuren, welche die Hybridisierung mit Ziel- bzw. Proben-Nukleinsäuren beeinflussen, können auf diese Weise berücksichtigt und rechnerisch korrigiert werden. Messungen, die mit Hilfe der Hybridisierung von Ziel- bzw. Proben-Nukleinsäuren mit erfindungsgemäßen Sonden-Nukleinsäuren durchgeführt werden, sind daher standardisierbar.

Die Fluoreszenzemission von jedem Probenpunkt des DNA-Arrays wird vorzugsweise durch Konfokale Laser-Scanning Mikroskopie bestimmt. Geräte zur Bestimmung der Fluoreszenzemission auf kleinen Flächen werden von einer Reihe von Hersteller angeboten und sind im Bereich der Biotechnologie Laborstandard [Cheung, V.G. et al., "Making and reading microarrays", Nature Genetics, vol. 21, Jan. 1999; Bowtell, D.D.L., "Options available - from start to finish - for obtaining expression data by microarray", Nature Genetics, vol. 21, Jan. 1999]. Zur Standardisierung der Meßergebnisse wird vor einer Hybridisierung die Fluoreszenz der immobilisierten Sonden-Nukleinsäuren bestimmt. Ist auf jedem Probenpunkt des Arrays eine Sonden-Nukleinsäure einer bestimmten Sequenzspezifität immobilisiert, die mit einer bestimmten Fluorophore markiert ist, so wird die Fluoreszenz durch Licht einer Wellenlänge angeregt, die dem Absorptionsmaximum (λ_{Abs.max}) diese Fluorophore entspricht, und bei einer Wellenlänge detektiert, die dem Emissionsmaximum (λ_{Em.max}) entspricht. Sind auf jedem Probenpunkt des DNA-Arrays n verschiedene Sonden-Nukleinsäuren unterschiedlicher Sequenzspezifität immobilisiert, die mit n verschiedenen, spektral unterscheidbaren Fluorophoren markiert sind, so wird die Fluoreszenz der n verschiedenen Fluorophoren durch Licht einer Wellenlänge angeregt, die dem Absorptionsmaximum (λ_{Abs.max}) dieser Fluorophoren entspricht, und bei einer Wellenlänge detektiert, die dem Emissionsmaximum (λ_{Em.max}) entspricht. Je nach verwendetem Gerät kann die Bestimmung der Fluoreszenz verschiedener Fluorophoren gleichzeitig oder nacheinander durchgeführt werden.

Die vorliegende Erfindung wird durch die nachfolgenden Beispiele näher erläutert.

### BEISPIEL 1:

Doppelt modifizierte Oligodesoxynukleotide, deren 5'-und 3'-Ende jeweils über einen C22-Spacer mit Fluoresceinisothiocyanat (FITC) bzw. einer Aminogruppe (NH₂) kovalent verbunden sind (Sequenz A: FITC-^{5'}gcccgcgcAATAGGGATGGCTCAACAgcgcgggc_{3'-}(C22)NH₂ und B: FITC-^{5'}gcccgcgcTTAGAGTGCAAAATGAAAGCGCCgcgcgggc_{3'-}(C22)NH₂) wurden in 100µl Kopplungspuffer (500 mM Na₂HPO₄, pH 8,5, 1mM EDTA) aufgenommen (Konzentration: 500 pmol/ml). Durch Inkubation von je 100 µl der Oligonukleotidlösung für 30 min bei RT (Raumtemperatur) in den Vertiefungen einer Mikrotiterplatte (Thermowell M PCR-Platte, Corning* Surface Technologies) inkubiert wurden die Oligonukleotide kovalent an die Oberfläche der Vertiefungen der gebunden wie in Tabelle 1 dargestellt:

### TABELLE 1

**Tabelle 1: Positionen der Proben-Nukleinsäuren A und B auf der Mikrotiterplatte**

| | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | A | - | - | - | A | - | - | - | A | - | - | - |
| **B** | - | - | B | - | - | - | B - | - | - | - | B | - |
| **C** | A | - | - | - | A | - | - | - | A | - | - | - |
| **D** | - | - | B | - | - | B | - | - | - | - | B | - |
| **E** | A | - | - | - | A | - | - | - | A | - | - | - |
| **F** | - | - | B | - | - | - | B | - | - | - | B | - |
| **G** | A | - | - | - | A | - | - | - | A | - | - | - |
| **H** | - | - | B | - | - | - | B | - | - | - | B | - |

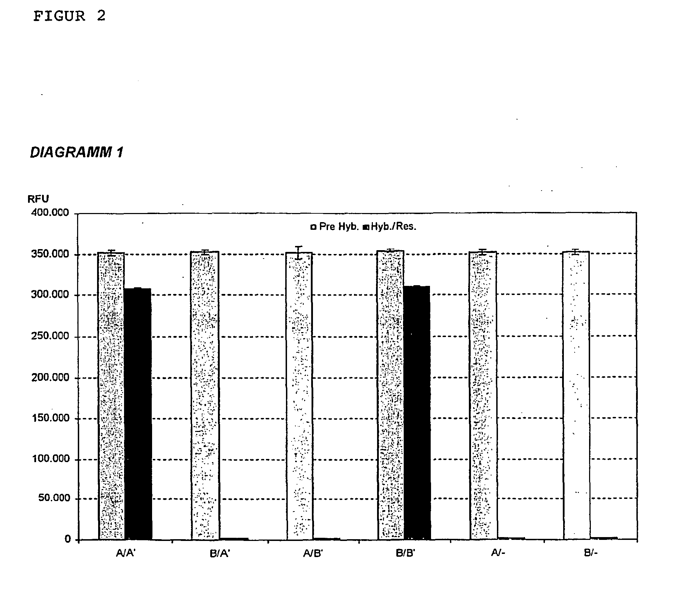

Anschließend wurden die Vertiefungen der Mikrotiterplatte 5 x mit 200 µl 10 mM Tris pH 8,0, 150 mM NaCl gewaschen. Die Fluoreszenzintensität der in den Vertiefungen der Mikrotiterplatte gebundenen Oligonukleotide wurde in einem Spektralfluorometer (Molecular Devices: Spectramax Gemini XS) bei der Anregungswellenlänge λ_{Abs.max} = 490nm und der Emissionswellenlänge λ_{Em.max} = 520 nm bestimmt. Die Daten sind in Tabelle 3 und Figur 2 (Diagramm 1) dargestellt:

### TABELLE 3

**Tabelle 3: Fluoreszenzintensität x 1000 der in den Vertiefungen einer Mikrotiterplatte kovalent gebundenen Oligonukleotide**

| | **1** | **2** | **3** | **4** | **5** | **6** | **7** | **8** | **9** | **10** | **11** | **12** |
|---|---|---|---|---|---|---|---|---|---|---|---|---|
| **A** | 352 | 0 | 0 | 0 | 348 | 0 | 0 | 0 | 351 | 0 | 0 | 0 |
| **B** | 0 | 0 | 351 | 0 | 0 | 0 | 356 | 0 | 0 | 0 | 351 | 0 |
| **C** | 356 | 0 | 0 | 0 | 355 | 0 | 0 | 0 | 348 | 0 | 0 | 0 |
| **D** | 0 | 0 | 350 | 0 | 0 | 0 | 353 | 0 | 0 | 0 | 349 | 0 |
| **E** | 348 | 0 | 0 | 0 | 350 | 0 | 0 | 0 | 356 | 0 | 0 | 0 |
| **F** | 0 | 0 | 356 | 0 | 0 | 0 | 354 | 0 | 0 | 0 | 352 | 0 |
| **G** | 351 | 0 | 0 | 0 | 352 | 0 | 0 | 0 | 352 | 0 | 0 | 0 |
| **H** | 0 | 0 | 353 | 0 | 0 | 0 | 351 | 0 | 0 | 0 | 358 | 0 |

Die Vertiefungen der Mikrotiterplatte wurden mit je 125 µl Hybridisierungslösung, die 0,1 mg/ml Salmon Sperm DNA sheared (Gibco BRULife Technologies); 0,5 mg/ml acetyliertes BSA (Gibco BRL/Life Technologies); 1X MES (100 mM MES, 1,0M NaCl, 20 mM EDTA, 0,01% Tween 20) enthielt, für 4 Stunden bei 60°C Prähybridisiert.
Nach Entfernen der Prähybridisierungslösung wurden 125 µl Hybridisierungslösung, die 1 nmol Proben-RNA-Oligonukleotide (Sequenzen A'= ^{5'}UGUUGAGCCAUCCCUAUU_{3'} bzw. B'= ^{5'}GGCGCUUUCAUUUUGCACUCUAA₃.) in 0,1 mg/ml Salmon Sperm DNA sheared (Gibco BRULife Technologies); 0,5 mg/ml acetyliertes BSA (Gibco BRL/Life Technologies); 1X MES (100 mM MES, 1,0M NaCl, 20 mM EDTA, 0,01% Tween 20) enthielten, in die Vertiefungen der Mikrotiterplatte gegeben. Der Ansatz ist in Tabelle 2 dargestellt:

### TABELLE 2

**Tabelle 2: Mit A', B' und - bezeichnete Bereiche der Mikrotiterplatte wurden mit den Proben-Nukleinsäuren A', B' bzw. Prähybridisierungslösung hybridisiert**

| | **1** | **2** | **3** | **4** | **5** | **6** | **7** | **8** | **9** | **10** | **11** | **12** |
|---|---|---|---|---|---|---|---|---|---|---|---|---|
| **A** | A' | A' | A' | A' | B' | B' | B' | B' | - | - | - | - |
| **B** | A' | A' | A' | A' | B' | B' | B' | B' | - | - | - | - |
| **C** | -A' | A' | A' | A' | B' | B' | B' | B' | - | - | - | - |
| **D** | A' | A' | A' | A' | B' | B' | B' | B' | - | - | - | - |
| **E** | A' | A' | A' | A' | B' | B' | B' | B' | - | - | - | - |
| **F** | A' | A' | A' | A' | B' | B' | B' | B' | - | - | - | - |
| **G** | A' | A' | A' | A' | B' | B' | B' | B' | - | - | - | - |
| **H** | A' | A' | A' | A' | B' | B' | B' | B' | - | - | - | - |

Der Ansatz wird für 20 min. auf 95°C erhitzt, im Verlauf einer Stunde auf 60 °C abgekühlt und für 16 Stunden bei 60°C hybridisiert.

Nach Entfernung der Proben-Nukleinsäurenlösung wurden die Vertiefungen der Mikrotiterplatte mit "nicht stringentem" Waschpuffer (6X SSPE; 0,01 % Tween 20) zehnmal für 5 Minuten bei 25°C und anschließend fünf mal mit "stringentem" Waschpuffer (100 mM MES; 0,1 M NaCl; 0,01 % Tween 20) für 5 Minuten bei 55°C gewaschen Nach dem Waschen wurden die Vertiefungen der Mikrotiterplatte mit 150 µl 1X Reaktionspuffer (NEB Buffer 3) für 10 Minuten bei 37°C äquilibriert und anschließend mit je 100 µl 1X Reaktionspuffer, der 2 Einheiten der Restriktionsendonuklease Aci1 (New England Biolabs) enthielt, für 1 Stunde bei 37°C inkubiert. Die Reaktion wurde durch Zugabe von 1/5 Volumen Stop-Lösung (0,5% w/v SDS, 50 mM EDTA) und durch Erhitzen der Mikrotiterplatte auf 75°C gestoppt. Anschließend wurden die Vertiefungen der Mikrotiterplatte 6 mal mit je 150 µl 1X TE-Puffer (10 mM Tris, 1 mM EDTA, pH=8,0) bei Raumtemperatur in gewaschen. Sämtliche nicht bei Raumtemperatur durchgeführten Inkubationen wurden in einem Biometra UNO™ Thermoblock mit Deckelheizung durchgeführt. Die Fluoreszenzintensität in den einzelnen Vertiefungen der Mikrotiterplatte wurde in einem Spektralfluorometer (Molecular Devices: Spectramax Gemini XS) bei der Anregungswellenlänge λ_{Abs.max} = 490nm und der Emissionswellenlänge λ_{Em.max} = 520 nm bestimmt. Die Daten sind in Tabelle 4 und Diagramm 1 (Figur 2) dargestellt:

### TABELLE 4

**Tabelle 4: Fluoreszenzintensität x 1000 der in den Vertiefungen einer Mikrotiterplatte kovalent gebundenen Oligonukleotide nach Hybridisierung und Restriktionsverdau**

| | **1** | **2** | **3** | **4** | **5** | **6** | **7** | **8** | **9** | **10** | **11** | **12** |
|---|---|---|---|---|---|---|---|---|---|---|---|---|
| **A** | 308 | 0 | 0 | 0 | 2,06 | 0 | 0 | 0 | 2,11 | 0 | 0 | 0 |
| **B** | 0 | 0 | 2,11 | 0 | 0 | 0 | 309 | 0 | 0 | 0 | 2,11 | 0 |
| **C** | 309 | 0 | 0 | 0 | 2,16 | 0 | 0 | 0 | 2,01 | 0 | 0 | 0 |
| **D** | 0 | 0 | 2,14 | 0 | 0 | 0 | 308 | 0 | 0 | 0 | 2,1 | 0 |
| **E** | 306 | 0 | 0 | 0 | 2,01 | 0 | 0 | 0 | 2,14 | 0 | 0 | 0 |
| **F** | 0 | 0 | 2,14 | 0 | 0 | 0 | 354 | 0 | 0 | 0 | 2,12 | 0 |
| **G** | 307 | 0 | 0 | 0 | 2,15 | 0 | 0 | 0 | 2,12 | 0 | 0 | 0 |
| **H** | 0 | 0 | 2,12 | 0 | 0 | 0 | 351 | 0 | 0 | 0 | 2,13 | 0 |

Das im Bereich der Vertiefungen 2A - 2H, 4A - 4H, 6A - 6H, 8A - 8H, 10A - 10H, 12A - 12H, 1B, 1D, 1F, 1 H, 3A, 3C, 3E, 3G, 5B, 5D, 5F, 5H, 7A, 7C, 7E, 7G, 9B, 9D, 9F, 9H, 11 B, 11D, 11E sowie 11G detektierbare Signal entspricht der Hintergrundfluoreszenz des Systems und wird von der im Bereich der Proben-Nukleinsäuren A und B detektierten Signale subtrahiert.

### BEISPIEL 2:

Die an unterschiedlichen Probenpunkten auf einer festen Matrix immobilisierten, mit Fluoresceinisothiocyanat (FITC) markierten Proben-Nukleinsäuren der Sequenz A: FITC-^{5'}gcccgcgcAATAGGGATGGCTCAACAgcgcgggc_{3'}, B: FITC-^{5'}gcccgcgcTTAGAGTGCAA-AATGAAAGCGCCgcgcgggc_{3'} und C: FITC-^{5'}gcccgcgcGTTTTTTTTTTTTGGTTTTTTTTTTTC-gcgcgggc_{3'} (Kontrolle; Bestimmung der Hintergrundfluoreszenz) werden mit 5 µg Proben-RNA, die aus K562-Zellen isoliert wurde, in 25 pM Kontroll-RNA, 0,1 mg/ml Salmon Sperm DNA sheared (Gibco BRULife Technologies); 0,5 mg/ml acetyliertes BSA (Gibco BRULife Technologies); 1X MES (100 mM MES, 1,0M NaCl, 20 mM EDTA, 0,01% Tween 20) für 16 Stunden bei 55°C hybridisiert. Nach Entfernung der Proben-Nukleinsäurenlösung wird der DNA-Array mit "nicht stringentem" Waschpuffer (6X SSPE; 0,01% Tween 20) zehn mal für 5 Minuten bei 25°C und anschließend fünfmal mit "stringentem" Waschpuffer (100 mM MES; 0,1 M Nacl; 0,01% Tween 20) für 5 Minuten bei 50°C gewaschen.

Nach dem Waschen wird der DNA-Array mit 1 X Reaktionspuffer für 10 Minuten bei 37°C äquilibriert. Anschließend wird der DNA-Array in 100 µl 1X Reaktionspuffer, der 2 Einheiten der Restriktionsendonuklease Aci1 enthält, für 1 Stunde bei 37°C inkubiert. Die Reaktion wird durch Zugabe von 1/5 Volumen Stop-Lösung (0,5 % w/v SDS, 50 mM EDTA) und durch Erhitzen des DNA-Arrays auf 75°C gestoppt. Anschließend wird der DNA-Array 4-8 mal bei Raumtemperatur in 1X TE (10 mM Tris, 1 mM EDTA, pH=8,0) gewaschen. Die Fluoreszenz der hybridisierten, auf dem DNA-Array verbliebenen Sonden wird durch Licht einer Wellenlänge λ_{Abs.max} = 490 nm angeregt und das bei einer Wellenlänge λ_{Em.max} = 520 nm emittierte Licht wird detektiert. Das im Bereich der Proben-Nukleinsäure C detektierbare Signal entspricht der Hintergrundfluoreszenz des Systems und wird von der im Bereich der Proben-Nukleinsäuren A und B detektierten Signale subtrahiert.

### BEISPIEL3:

Die mit den Fluorophoren FITC (λ_{Abs.max}= 490 nm, λ_{Em.max} = 520 nm), Cascade Blue (λ_{Abs.max} = 400 nm, λ_{Em.max} = 420 nm), und BODIPY _{TR14} (λ_{Abs.max} = 595 nm, λ_{Em.max} = 625 nm), markierten Proben-Nukleinsäuren der Sequenz A: (FITC)-^{5'}gcccgcgcAATAGGGATGGCTCAACA-gcgcgggC₃, B: (Cascade Blue)-^{5'}gcccgcgcTTAGAGTGCAAAATGAAAGCGCCgcgCgggc₃, und C: (BODIPY TR14)-^{5'}gcccgcgcTTTCTCTACCTCCTCACATTGTGgcgcgggc_{3'} werden auf einer definierten Fläche A (Probenpunkt A) gemeinsam immobilisiert. Die SOnden-Nukleinsäuren D: (FITC)-^{5'}gcccgcgcGTTTTTTTTTTTTGGTTTTTTTTTTTC-gcgcgggc_{3'}, E: (Cascade Blue)-^{5'}gcc-cgcgcGTTTTTTTTTTGGTTTTTTTTTTTC-gcgcgggc₃- und F: (BODIPY TR14)-^{5'}gcccgcgc-GTTTTTTTTTTTTGGTTTTTTTTTTTC-gcgcgggc₃', die als Kontrolle zur Bestimmung der Hintergrundfluoreszenz eingesetzt werden, werden auf einer weiteren definierten Fläche B (Probenpunkt B) gemeinsam immobilisiert. Der DNA-Array wird mit 0,1 µg Proben-RNA (cRNA), welche die Sequenzen D' = ^{5'}UGUUGAGCCAUCCCUAUU_{3'}, E' = ^{5'}GGCGCUUUCAUUUUGCACUCUAA₃. und F' = ^{5'}CACAAUGUGAGGAGGUAGAGAAA₃. enthält, in 25 pM Kontroll-RNA, 0,1 mg/ml Salmon Sperm DNA sheared (Gibco BRL/Life Technologies); 0,5 mg/ml acetyliertes BSA (Gibco BRULife Technologies); 1X MES (100 mM MES, 1,0M NaCl, 20 mM EDTA, 0,01 % Tween 20) für 16 Stunden bei 55°C hybridisiert. Nach Entfernung der Proben-Nukleinsäurenlösung wird der DNA-Array mit "nicht stringentem" Waschpuffer (6X SSPE; 0,01 % Tween 20) zehnmal für 5 Minuten bei 25°C und anschließend fünfmal mit "stringentem" Waschpuffer (100 mM MES: 0,1 M NaCl; 0,01 % Tween 20) für 5 Minuten bei 50°C gewaschen.

Nach dem Waschen wird der DNA-Array mit 1X Reaktionspuffer für 10 Minuten bei 37°C äquilibriert. Anschließend wird der DNA-Array in 100 µl 1X Reaktionspuffer, der 2 Einheiten der Restriktionsendonuklease Aci1 enthält, für 1 Stunde bei 37°C inkubiert. Die Reaktion wird durch Zugabe von 1/5 Volumen Stop-Lösung (0,5 % w/v SDS, 50 mM EDTA) und durch Erhitzen des DNA-Arrays auf 75°C gestoppt. Anschließend wird der DNA-Array 4 - 8 mal bei Raumtemperatur in 1X TE (10 mM Tris, 1 mM EDTA, pH=8,0) gewaschen. Die Fluoreszenz der hybridisierten, auf dem DNA-Array verbliebenen und mit den Fluorophoren FITC, Cascade Blue oder BODIPY TR14 markierten Sonden wird durch Licht der Wellenlängen λ_{Abs.max} = 490 nm, λ_{Abs.max} = 400 nm bzw. λ_{Abs.max} = 595 nm angeregt und das bei einer Wellenlänge λ_{Em.max} = 520 nm, λ_{Em.max} = 420 nm bzw, λ_{Em.max} = 625 nm emittierte Licht wird detektiert. Die im Bereich des Probenpunktes B detektierbaren Signale entsprechen der Hintergrundfluoreszenz des Systems und werden von der im Bereich des Probenpunktes A detektierten Signalen subtrahiert.

## Patentansprüche

1. Verfahren zum Nachweis von Zielnukleinsäure durch Hybridisierung, **dadurch gekennzeichnet, daß** in dem Verfahren
a) eine Hybridisierung mit wenigstens einer Sonden-Nukleinsäure durchgeführt wird, die mit einem Ende an einer festen Phase gebunden ist, wobei die Sonden-Nukleinsäure die Zielnukleinsäuresequenz oder eine dazu komplementäre Sequenz aufweist, die am 3'-Ende von einer kurzen Nukleinsäuresequenz und am 5'-Ende von einer zu dieser Nukleinsäure komplementären Nukleinsäuresequenz flankiert ist, die einen DNA-Doppelstrang und **dadurch** ein Spaltmodul ausbilden, das eine Erkennungssequenz für eine Restriktionsendonuklease enthält und von der Restriktionsendonuklease gespalten werden kann, und diese Sonden-Nukleinsäure am anderen Ende eine Markierung aufweist,
b) wenigstens eine Behandlung mit der Restriktionsendonuklease durchgeführt wird und
c) der Anteil der Markierung bestimmt wird, der nach Schritt b) an der festen Phase gebunden ist.

2. Verfahren nach Anspruch 1, **dadurch gekennzeichnet, daß** in einem Verfahrensansatz mehrere Sonden-Nukleinsäuren gleichzeitig eingesetzt werden, die voneinander verschiedene Zielsequenzen beinhalten.

3. Verfahren nach einem der Ansprüche 1 oder 2, **dadurch gekennzeichnet, daß** in einem Verfahren mehrere Sonden-Nukleinsäuren eingesetzt werden, die voneinander verschiedene Spaltmodule aufweisen, die von verschiedenen Restriktionsendonukleasen gespalten werden können.

4. Verfahren nach einem der Ansprüche 1-3, **dadurch gekennzeichnet, daß** die Sonden-Nukleinsäuren mehrere voneinander verschiedene Markierungen aufweisen.

5. Verfahren nach Anspruch 4, **dadurch gekennzeichnet, dass** es sich bei den Markierungen um Fluorophore und/oder Teile eines Bindungspaares handelt.

## Claims

1. A method for detecting target nucleic acid by means of hybridization, **characterized in that**, in the method,
a) a hybridization is carried out using at least one probe nucleic acid which is bound by one of its ends to a solid phase, with the probe nucleic acid possessing the target nucleic acid sequence, or a sequence which is complementary to it, which is flanked, at its 3' end, by a short nucleic acid sequence and, at its 5' end, by a nucleic acid sequence which is complementary to this nucleic acid, which nucleic acid sequences form a DNA double strand and thereby a cleavage module which contains a recognition sequence for a restriction endonuclease and which can be cleaved by the restriction endonuclease, and with this probe nucleic acid possessing a label at its other end,
b) at least one treatment with the restriction endonuclease is carried out, and
c) the proportion of the label which is bound to the solid phase after step b) is determined.

2. The method as claimed in claim 1, **characterized in that**, in one methodological setup, several probe nucleic acids, which contain target sequences which differ from each other, are used simultaneously.

3. The method as claimed in claim 1 or 2, **characterized in that** use is made, in one procedure, of several probe nucleic acids which possess cleavage modules which differ from each other and which can be cleaved by different restriction endonucleases.

4. The method as claimed in one of claims 1-3, **characterized in that** the probe nucleic acids possess several labels which differ from each other.

5. The method as claimed in claim 4, **characterized in that** the labels are fluorophores and/or parts of a binding pair.

## Revendications

1. Procédé de mise en évidence d'un acide nucléique cible par hybridation, **caractérisé en ce que** dans le procédé,
a) on réalise une hybridation avec au moins un acide nucléique sonde, qui, à une extrémité est relié à une phase solide, l'acide nucléique sonde comportant la séquence d'acides nucléiques cible ou une séquence complémentaire de celle-ci, qui est flanquée à l'extrémité 3' d'une séquence d'acides nucléiques courte et à l'extrémité 5' d'une séquence d'acides nucléiques complémentaire à ces acides nucléiques, lesquels forment un ADN double brin et de ce fait un module de clivage qui contient une séquence de reconnaissance d'une endonucléase de restriction et qui peut être clivé par l'endonucléase de restriction, cet acide nucléique sonde présentant à l'autre extrémité une substance de marquage,
b) on effectue au moins un traitement au moyen de l'endonucléase de restriction, et
c) on détermine la fraction de la substance de marquage qui s'est liée à la phase solide après l'étape b).

2. Procédé selon la revendication 1, **caractérisé en ce que** l'on utilise simultanément plusieurs acides nucléiques sondes dans une procédure opératoire, lesquels contiennent des séquences cibles qui diffèrent les unes des autres.
3) Procédé selon la revendication 1 ou 2, **caractérisé en ce que** l'on utilise plusieurs acides nucléiques sondes dans un procédé, qui présentent des modules de clivage qui diffèrent les uns des autres et qui peuvent être clivés par des endonucléases de restriction différentes.
4) Procédé selon l'une des revendications 1 à 3, **caractérisé en ce que** les acides nucléiques sondes présentent une pluralité de substances de marquage qui diffèrent les unes des autres.
5) Procédé selon la revendication 4, **caractérisé en ce que** les substances de marquage sont des fluorophores et/ou des éléments d'une paire de liaison.
